# EUROPEAN PATENT APPLICATION

(11) **EP 4 768 049 A1**
(43) Date of publication of application: **01.07.2026**
(21) Application number: 24383469.4
(22) Date of filing: 27.12.2024
(51) Int. Cl.: A61L 9/22, F24F 8/30, F24F 3/16, F24F 13/08

(54) **ENRICHED AIR DELIVERY DEVICE**

(71) Applicant: Biow Exposomics, S.L., 33211 Gijon (Asturias) (ES)
(72) Inventor: LLANA GARCÍA, Pedro Luis, 33211 Gijon (Asturias) (ES)
(74) Representative: Herrero & Asociados, S.L.

(57) **Abstract**

An enriched air delivery device comprises an upper casing (10); a lower casing (20) provided with ventilation grids (21); a chamber (30) located between both casings (10, 20), which houses air inside it; at least one atmospheric cold plasma generator (40) housed between both casings (10, 20), and configured to emit negatively charged ions onto the air in the chamber (30), thus creating enriched air; and an external device (50) connected by wiring to the at least one atmospheric cold plasma generator (40), for its electrical supply; such that in an operating state of the device (1), and applied to the skin of a user, the enriched air existing in the chamber (30) is released to the outside through the ventilation grids (21) of the lower casing (20), such that said enriched air is naturally absorbable by the user's own skin.

## Description

### OBJECT OF THE INVENTION

The present invention belongs to the field of air conditioning systems, as well as dermatological devices for skin care.

The object of the present invention is a portable enriched air delivery device for easy manual handling, such that through an air chamber enriched with bioavailable ions, the user's skin, muscle or fat can naturally absorb the energy accumulated in said air chamber by electromagnetism and permeability through the skin, resulting in multiple benefits for the health and quality of life of users. All of this in a harmless manner, without the risk of suffering damage from electric discharge or plasma, and without the risk of overdosing for users.

### BACKGROUND OF THE INVENTION

Nowadays, air conditioning systems designed to improve the air quality in closed spaces are widely known. These devices have evolved significantly to meet the growing demand for healthy and pollutant-free environments. The need to improve air quality has become increasingly important in domestic, commercial and industrial environments, as clean air contributes to the reduction of respiratory diseases, improves general well-being and increases productivity in work spaces.

Among the advanced technologies for air and skin treatment, the use of cold atmospheric plasma, known in English as "Cold Atmospheric Plasma" (CAP), stands out. Cold plasma is a gas-like state of matter obtained by ionizing a gas at low temperature, which generates charged particles (ions, electrons and free radicals) at low temperatures, allowing it to be applied directly to the skin without damaging it, and which have the ability to neutralize microorganisms, eliminating viruses, bacteria and other pathogens without the need for aggressive chemicals. This plasma is generated through devices that use an electric current to ionize the air at room temperature. Due to these properties, CAP has generated considerable interest in sterilization, surface treatment and microbiological control applications.

The use of CAP pens is also known, traditionally limited to very specific applications in the field of medicine, such as surgical procedures, tumor treatment and healing of difficult-to-treat wounds. In these cases, devices called "torch" or plasma torches are used, which concentrate cold plasma in very small areas with a high intensity, suitable for high-precision medical applications. However, these applications involve certain risks due to the high concentration of energy and the direct exposure of sensitive tissues, which requires trained personnel and strict safety measures.

Years of biomedical research on the delivery of electrons through the skin, and numerous scientific studies have shown that enriched air, obtained from plasma generators, allows the activation of the signaling pathways that take place in the intercellular matrix to facilitate the function of stem cells in the regeneration of tissues and muscles. This is especially noticeable in the most exposed tissues, such as skin or hair, with the consequent improvements obtained, preventing cardiovascular diseases, strengthening the immune system, reducing the production of free radicals and avoiding oxidative stress, a key factor in the fight against aging. ("Removal of Environmental Nanoparticles Increases Protein Synthesis and Energy Production in Healthy Humans", Eduardo Antuña et al., February 2022).

Indeed, it is widely known that plasma inducers or generators modify the exposome at all levels, where in the context of skin care, the term "exposome" would encompass all those external and internal factors to which an individual is exposed throughout his or her life, and which contribute to skin aging, beyond genetics. In this way, through plasma generators that carry out this modification of the exposome, it is possible to eliminate contaminating particles in the environment closest to the cells, facilitating the natural processes of cellular regeneration to take place.

Despite the success of these applications in the medical field, the potential of cold plasma for air treatment in non-medical and general-use environments has been scarcely explored. The main limitation lies in the lack of technologies that allow the safe and efficient use of CAP with a controlled and uniform distribution, avoiding the risks associated with medical applications. Therefore, there is a need to develop devices that take advantage of the benefits of CAP for air conditioning, without the risks of intense exposure, and that allow the range of applications to be extended to dermatological levels and/or for the care of the skin, muscle or fat of the users.

### DESCRIPTION OF THE INVENTION

The present invention solves the aforementioned drawbacks by providing an enriched air delivery device for comfortable, easy and safe use on a user's skin, with notable benefits for the health and quality of life of users, both in the skin and in muscle tissue and even body fat. Furthermore, the application of the device described herein on the human body has been shown to have significant benefits for abdominal volume, the genitourinary system, the digestive system, hair and even the resting of users, as will be explained later.

The enriched air delivery device of the invention is defined in the independent claim. The dependent claims include other features, which are optional.

The enriched air delivery device comprises: an upper casing; a lower casing provided with ventilation grids; a chamber located between both casings, which houses air inside; at least one cold atmospheric plasma generator (CAP) housed between both casings, and configured to emit negatively charged ions (anions) into the air in the chamber, thus creating enriched air; and external equipment connected by wiring to the at least one cold atmospheric plasma generator, for its electrical supply.

Thus, in an operating state of the device, and applied to the skin of a user, the enriched air in the chamber is released to the outside through the ventilation grids of the lower casing, such that said enriched air is naturally absorbable by the user's own skin, muscle or fat.

More specifically, the device of the invention works at the mitochondrial level (source of energy for cells), in terms of nanotoxicity and biotoxicity, increasing the production of energy in the form of ATP (Adenosine Triphosphate molecule), energy carriers, reducing cellular wear.

In this way, through the air chamber enriched with bioavailable ions, the user's skin, muscle or fat can naturally absorb the energy accumulated in said air chamber by electromagnetism and permeability through the skin. All this in a way that is harmless to the user, without risks of electric or plasma discharge, and without risks of overdose.

It should be noted here that the term "bioavailable ions" refers to electrically charged atoms or molecules that are in a form that is accessible and usable by the body, especially in topical or internal treatments. These ions are able to interact with cells and tissues effectively, facilitating processes such as healing, pH regulation and neutralization of free radicals. More specifically, in the context of skin care, bioavailable ions can improve nutrient absorption and strengthen the skin barrier, promoting cell regeneration and hydration.

At this point, it should be noted that the plasma generator does not discharge directly onto the skin, but instead emits into the air in the chamber, which is enriched with reactive oxygen and nitrogen species (bioavailable ions), making this air make these components available to the user's body, which will absorb them or not depending on the previous electrical charge of the skin. This is important as it avoids all types of risks from electric, plasma discharge and overdosing. Essentially, the delivery device of the invention makes it possible to create an enriched air chamber at a distance such that the user's skin absorbs what it needs.

In this way, the device of the invention allows the enriched air to be focused with CAP plasma generators, creating a film for being applied on the skin, positioning the generation source very close, but without any damage or risk to the user. More specifically, using the enriched air delivery device of the present invention it is possible to generate up to 2 million anions/ cm³, applying it close to the user's skin (approximately 1 cm), thus maximizing the use of those 2 million anions/ cm³ emitted in a localized manner.

Furthermore, the device of the invention has reduced dimensions, allowing easy manual handling by the user, without requiring the intervention of a specialized professional, giving the device a portable character and domestic use.

As already mentioned, the device of the present invention does not involve any risk for the user, since both the intensity and the distance of application between the energy source and the application surface of the user's skin are precisely measured, providing a completely harmless device, while still stimulating the epidermis, the lower layers of the skin, the muscles and even the blood and organs. Indeed, the device of the invention also has advantageous effects on the user's muscles, allowing abdominal distension to be reduced, and even increasing muscular tension in the pelvic floor, thus preventing urine loss. Likewise, the application of the device on the abdomen of a user has been shown to have beneficial effects on digestion and the genitourinary system.

Furthermore, it has been provided that the enriched air delivery device described herein can be attached to an extendable support (lamp type or the like) used at night, while users sleep. This mode of use of the device has been shown to have additional advantageous effects, such as:
- better rest, promoting deeper and more continuous sleep.
- better hair, obtaining thicker and fuller hair.

Therefore, the device of the present invention provides a solution for the creation of an air chamber enriched with bioavailable ions, so that the user can absorb the energy accumulated in said air chamber by electromagnetism and permeability through the skin, allowing the user to benefit from the advantageous effects mentioned above. Indeed, this special combination of bioavailable ions and atmospheric cold plasma represents a promising therapeutic option for the treatment of various skin, muscle or fat conditions, with the potential to improve the appearance and regeneration of the skin in a safe and effective manner, obtaining important beneficial effects for the health and quality of life of users (skin, abdominal volume, genitourinary system, digestive system, hair and rest).

### DESCRIPTION OF THE DRAWINGS

In order to complement the description being made and to assist in a better understanding of the features of the invention, in accordance with a preferred example of its practical implementation, a set of drawings is included as an integral part of said description, in which, for illustrative and non-limiting purposes, the following has been represented:
Figure 1.- Shows a bottom perspective view of the enriched air delivery device of the present invention.
Figure 2.- Shows a top perspective view of the device of the invention.
Figure 3.- Shows an exploded view of the device object of invention.
Figures 4A - 4C.- Show the elevation, profile and bottom plan views of the device of the invention.
Figures 5A, 5B.- Show a pair of views of the device of the invention, configured for coupling to an extendable support, according to a second preferred embodiment.
Figures 6A, 6B.- Show a pair of views of the device of the invention, according to a third preferred embodiment.
Figures 7A, 7B.- Show a pair of views of the device of the invention, according to a fourth preferred embodiment.
Figures 8A, 8B.- Show a pair of views of the device of the invention, according to a fifth preferred embodiment.

### PREFERRED EMBODIMENT OF THE INVENTION

An example of a preferred embodiment is described below with reference to the figures cited above, without limiting or reducing the scope of protection of the present invention.

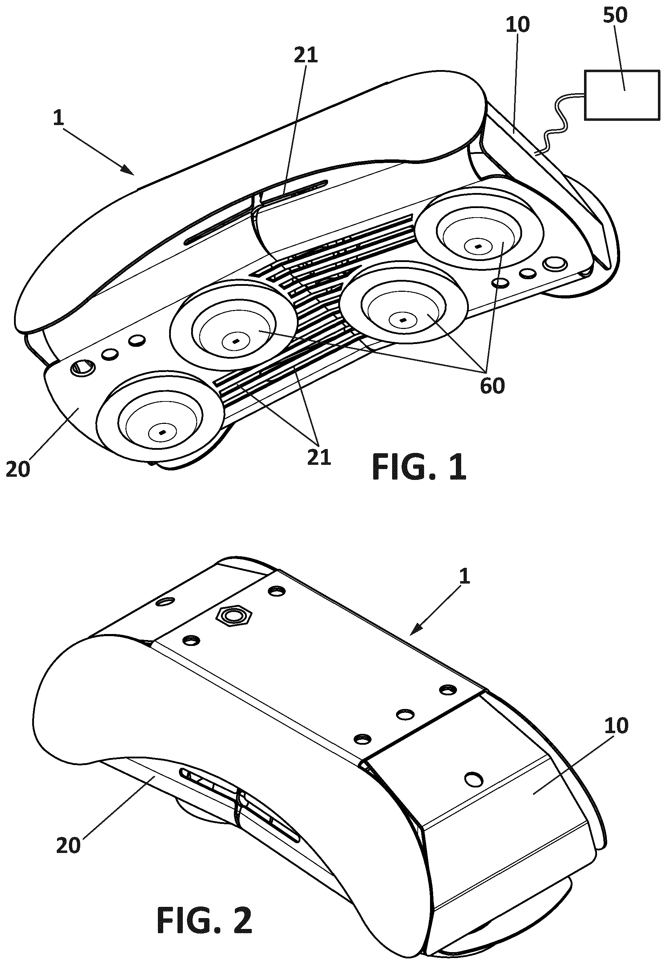

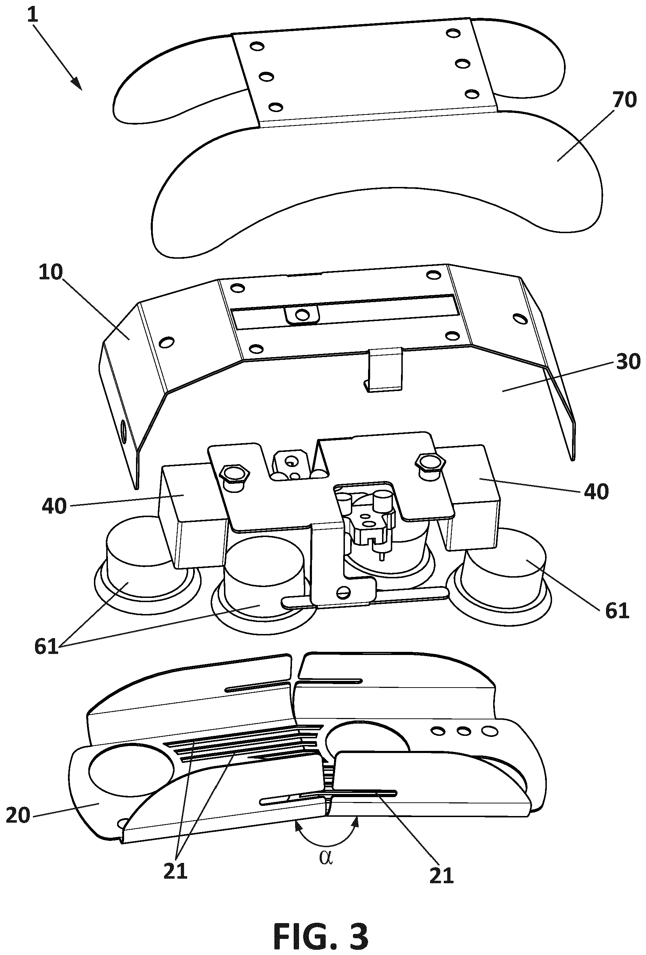

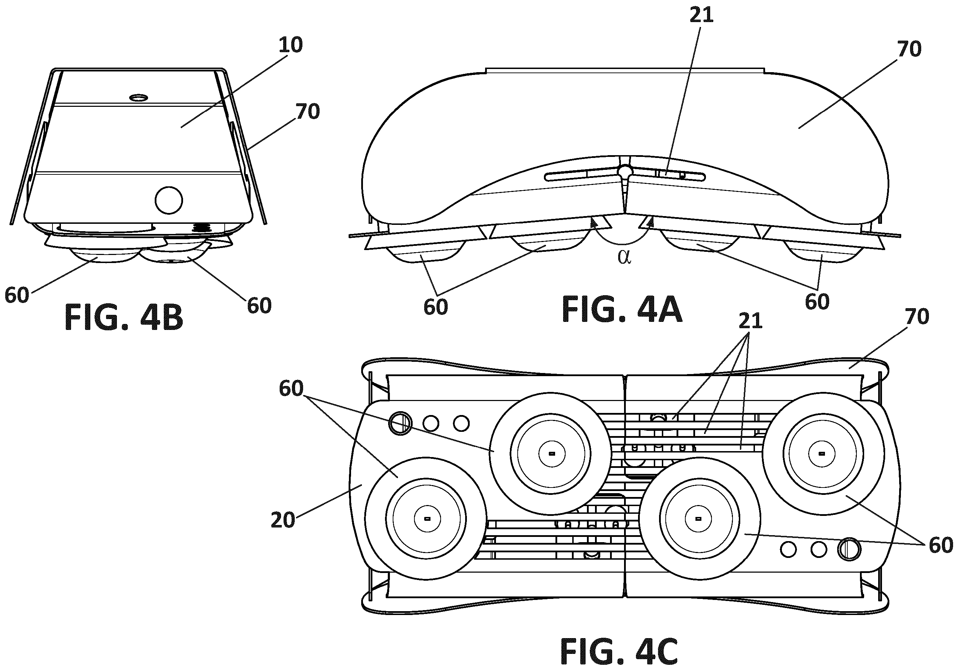

According to a preferred embodiment, shown in figures 1 and 2, the enriched air delivery device (1) comprises:
- an upper casing (10);
- a lower casing (20) provided with ventilation grids (21) clearly shown in figures 1 and 4C;
- a chamber (30) located between both housings (10, 20), which houses air inside;
- two atmospheric cold plasma generators (40), shown in Figure 3, housed between both housings (10, 20), and configured to emit negatively charged ions into the chamber air (30), thus creating enriched air;
- an external equipment (50) connected by wiring to the atmospheric cold plasma generators (40), for its electrical supply; and
- wheels (60), shown in figures 1 and 4, in this case as bearings or rotating spherical balls, to facilitate the sliding of the device (1).

In an operating state of the device (1), and applied to the skin of a user, the enriched air existing in the chamber (30) is released to the outside through the ventilation grids (21) of the lower casing (20), such that said enriched air can be absorbed naturally (on demand) by the user's own skin, that is, the skin absorbs this enriched air according to its needs.

It should be noted that the device (1) of the invention does not deliver enriched air under pressure, but rather a micro-cloud is created in the chamber (30), rich in bioavailable ions that the skin will absorb naturally due to the difference in charge. In this way, the safety of the device is ensured, since absorption is not forced, it is simply made available to the skin cells.

In figure 3 it can be seen that the wheels (60) are partially housed in internal boxes (61) located between both casings (10, 20). In fact, in figure 1 it can be seen that the central area with the largest perimeter of each of the wheels (60) coincides with the internal perimeter edge of the boxes (61). This favors the turning of the wheels with little effort, with a smooth movement and precise rolling, at the same time that it avoids obstructions or blockages of the wheels, preventing the insertion of any foreign element inside the box (61).

In this regard, it should be clarified that the wheels (60) not only facilitate the sliding of the device (1) over the user's skin, but also allow a precise safety distance to be guaranteed between the plasma generators (40) and the application surface of the skin area to be treated.

However, it has been foreseen that the device (1) of the invention can be built without wheels (60), for example, by means of a ski-like shape, such that they guarantee the appropriate distance for its use in a static manner, without movement, either by means of plastic or padded separators. In this sense, it should be noted that the use of the device (1) of the invention in a static manner on a specific area of the human body has also been shown to have multiple benefits, both in the digestive system, in intestinal transit, as well as in the genitourinary system.

According to the preferred embodiment shown in Figures 3 and 4A, the lower casing (20) is formed by two halves arranged with respect to each other at a convergent angle (α) of less than 180°. This feature favors the focusing of the enriched air, which is released through the ventilation grids (21) of the lower casing (20), onto a specific area of the user's skin; at the same time, it allows for optimum adaptation and coupling to the special natural curvatures of the human body.

In Figure 3 it can be seen that, according to this preferred embodiment, the lower casing (20) has a U-shaped configuration, with two longitudinal side walls located in the vertical plane. This allows for greater structural rigidity to the assembly, while facilitating its coupling with the upper casing (10). Furthermore, in Figures 1, 3 and 4A it can be seen that each of the two longitudinal side walls of the lower casing (20) has at least one additional ventilation grid (21). This favors an optimal release of the enriched air coming from the interior of the chamber (30), for a complete and homogeneous absorption by the specific area of skin being treated at any time.

On the other hand, it has been provided that the upper casing (10) may have an external layer of skin (70), shown in figures 3, 4A, 4B, which in addition to promoting the grip of the device (1), with a firmer and non-slip hold, provides a softer and more pleasant user experience to the touch, providing the device (1) with a more elegant and attractive aesthetic finish.

As can be seen in Figures 2 and 4A, the device (1) of the invention has an ergonomic configuration, wherein both the upper casing (10) and the lower casing (20) have blunt and rounded edges and corners. This allows for greater comfort and ease of use, with a more natural and comfortable grip, reducing hand fatigue; greater safety without the risk of cuts or injuries during handling; and greater durability of the device (1), reducing wear and damage to the edges, prolonging the useful life of the device (1).

Furthermore, as already mentioned, the device (1) of the invention has reduced dimensions, allowing easy manual handling by the user, without requiring the intervention of a specialized professional, providing the device with a portable character and domestic use. This allows large areas of the user's body to be treated without effort (for example, the abdomen), but also to focus on specific areas (for example, the pelvis, for treatments against menstrual pain, treatments for recovery from injuries in arms and legs, etc.).

Although not shown in the figures, it has been provided that the enriched air delivery device (1) of the present invention may additionally comprise collagen lamps, located inside the device (1), between both casings (10, 20). This makes it possible to reduce age spots, sun spots and other skin discolorations. Preferably, said collagen lamps emit red light of 630 nanometers, which makes it possible to increase the transmission of energy between the molecules that absorb the light. Thus, these already activated molecules favor the oxygenation and detoxification of the skin.

Similarly, it has also been provided that the device (1) may incorporate heating means and/or vibration means, located inside the device (1), between both casings (10, 20). In this way, improved skin stimulation is obtained, wherein heat and vibration activate blood circulation, improving oxygenation of the skin, helping to relax the muscles, reducing tension and promoting more rested skin.

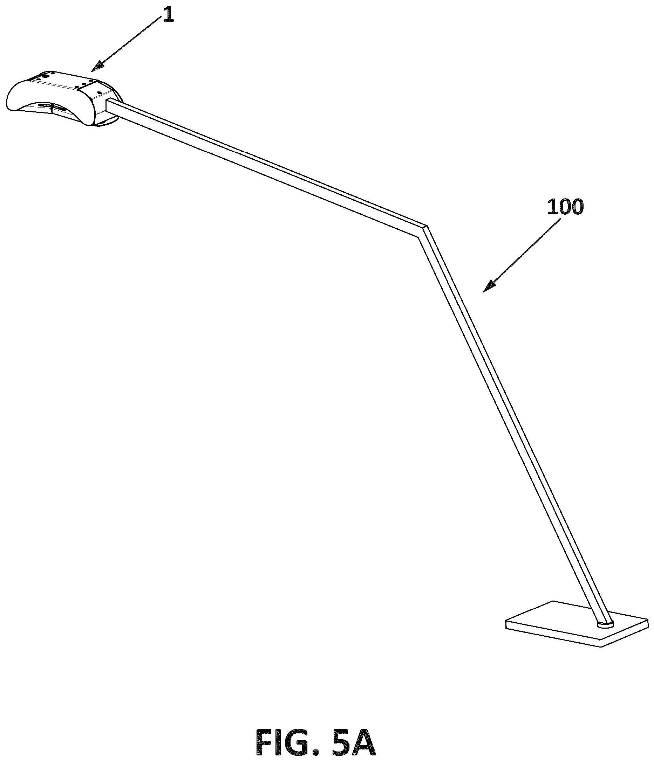

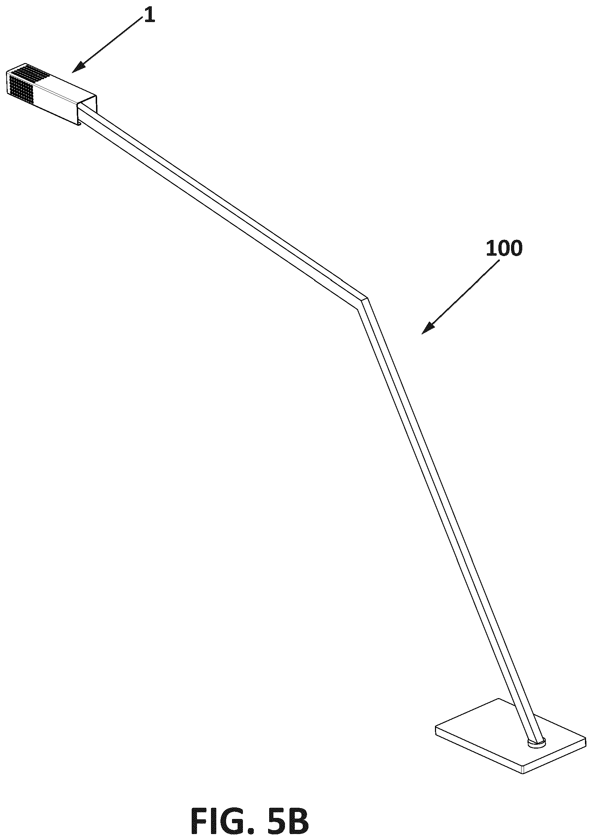

According to a second preferred embodiment of the invention, shown in Figures 5A and 5B, the enriched air delivery device (1) can be attached to an extendable lamp-type support (100), intended to be used at night, while users sleep. This mode of use of the device (1) of the invention allows for better rest, promoting deeper and more continuous sleep; at the same time as better hair, obtaining thicker and fuller hair. In this second preferred embodiment, while in Figure 5A the device (1) has an ergonomic configuration, where both the upper casing (10) and the lower casing (20) have blunt and rounded edges and corners; in Figure 5B the device (1) has a rectangular prism configuration, with a plurality of holes made in the vicinity of its distal end, for the release of the enriched air.

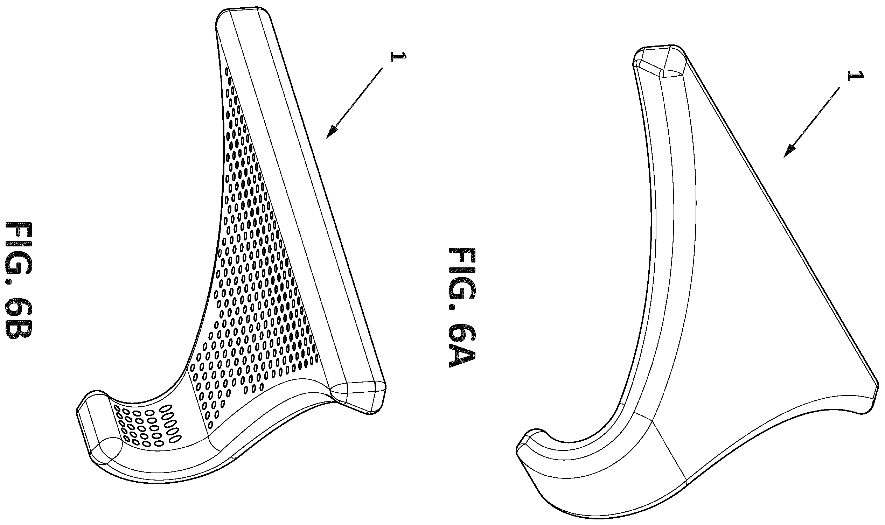

According to a third preferred embodiment of the invention, shown in Figures 6A and 6B, the enriched air delivery device (1) has a quasi-triangular shape, intended to be used on the area corresponding to the user's genito-urinary system, which includes the genitals, the reproductive organs and the urinary organs. More specifically, according to this third preferred embodiment, the device (1) has a convex arc section at one of its vertices, intended to cover or wrap the genitals, being located on the user's inner thigh. As can be seen in figure 6B, the inner face of the device (1) has a plurality of holes for the release of the enriched air. The device (1) according to this embodiment allows relief of the symptoms of menopause in women, helping to reduce hot flashes, night sweats and other common symptoms of menopause. In addition, the device (1) according to this third embodiment has shown to obtain good results both in increasing the muscular tension of the pelvic floor, and helping to prevent urine loss.

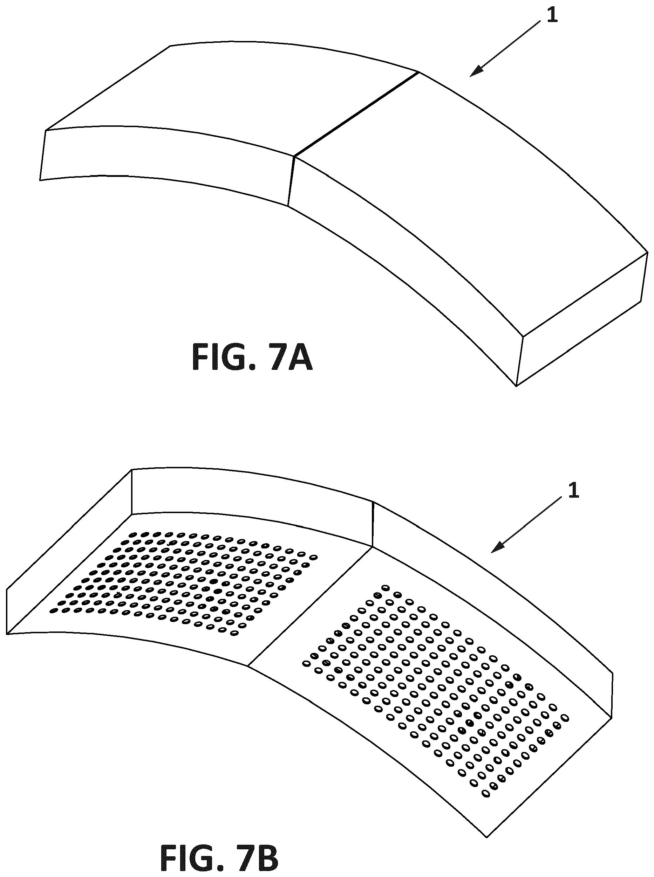

According to a fourth preferred embodiment of the invention, shown in Figures 7A and 7B, the enriched air delivery device (1) has a flattened rectangular configuration that defines a convex curvature, intended to be located on the user's abdomen. As can be seen in Figure 7B, the internal face of the device (1) has a plurality of holes for the release of the enriched air. It has been proven that the device (1) according to this embodiment allows obtaining multiple benefits both at the level of the digestive system and in intestinal transit, being also useful in therapies for the reduction of localized fat and abdominal distension.

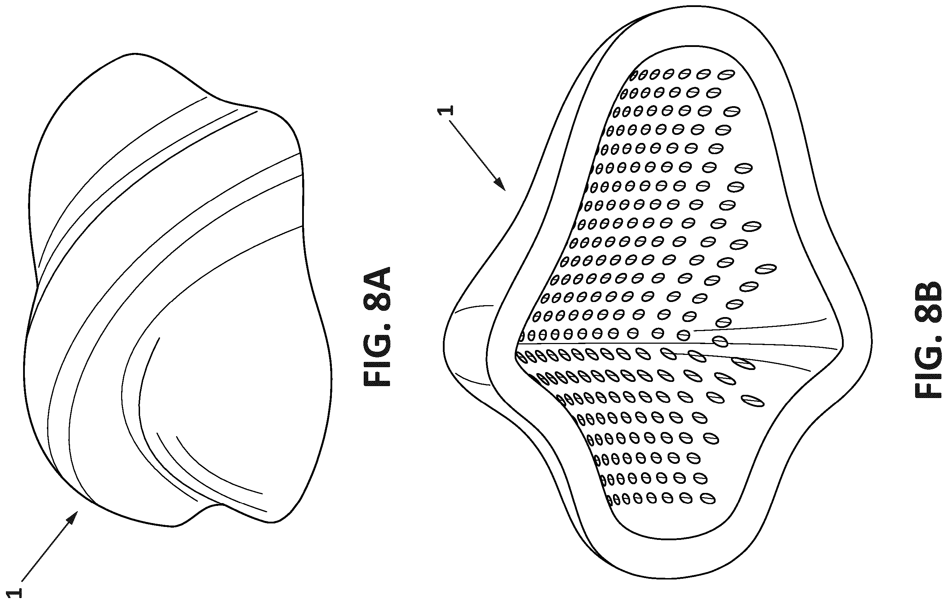

According to a fifth preferred embodiment of the invention, shown in Figures 8A and 8B, the enriched air delivery device (1) has a mask configuration, specifically a face mask shape intended to be placed on the user's face, with respective curvatures and extensions to cover the mouth and nose. As can be seen in Figure 8B, the inner face of the device (1) has a plurality of holes for the release of the enriched air. The device (1) according to this fifth embodiment allows obtaining important facial beauty benefits, improving the firmness and elasticity of the skin, helping to achieve a brighter and healthier complexion, and contributing to reducing the visible signs of aging.

Some of the advantageous effects obtained from the enriched air delivery device (1) of the invention are listed below:
On the skin:
   - Antimicrobial properties: elimination of bacteria, fungi and viruses, for the treatment of wound infections, dermatitis or even acne.
   - Stimulation of healing: acceleration of wound healing, for a better and faster recovery of damaged skin.
   - Cell regeneration: proliferation of fibroblasts and keratinocytes, useful for the treatment of burns and injuries that require rapid tissue regeneration.
On muscle tissues:
   - Muscle regeneration and repair: promoting cell proliferation and the formation of new blood vessels (angiogenesis), facilitating faster recovery after muscle damage or surgical interventions.
   - Anti-inflammatory properties: helping to reduce inflammation in injured muscles.
   - Improved oxygen supply: by stimulating the formation of new blood vessels (angiogenesis), which improves the flow of blood and oxygen to the muscles, useful for the treatment of damaged muscles or in rehabilitation processes.
On body fat:
   - Free radical-induced lipotoxicity: generation of reactive oxygen and nitrogen species (ROS and RNS), which can induce oxidative damage in various cell types, including adipocytes (fat cells). Useful in therapies for the reduction of localized fat.
   - Anti-inflammatory effects on adipose tissue: In the context of obesity-related diseases, such as chronic inflammation of adipose tissue, it reduces inflammation and improves the metabolic function of fatty tissue.
   - Stimulation of lipolysis: it can induce lipolysis (fat breakdown) by influencing the cellular mechanisms that regulate fat storage and release. Useful for the treatment of cellulite.

## Claims

1. Enriched air delivery device (1), **characterized in that** it comprises:
- an upper casing (10);
- a lower casing (20) provided with ventilation grids (21);
- a chamber (30) located between both casings (10, 20), which houses air inside it;
- at least one atmospheric cold plasma generator (40) housed between both casings (10, 20), and configured to emit negatively charged ions onto the air in the chamber (30), thus creating enriched air; and
- external equipment (50) connected by wiring to the at least one atmospheric cold plasma generator (40), for its electrical supply;
such that in an operating state of the device (1), and applied to the skin of a user, the enriched air existing in the chamber (30) is released to the outside through the ventilation grids (21) of the lower casing (20), such that said enriched air is naturally absorbable by the user's own skin.

2. Enriched air delivery device (1) according to claim 1, **characterized in that** it additionally comprises wheels (60) to promote the sliding of the device (1).

3. Enriched air delivery device (1) according to claim 2, **characterized in that** the wheels (60) are partially housed in internal boxes (61) located between both casings (10, 20).

4. Enriched air delivery device (1) according to claim 1, **characterized in that** the upper housing (10) has an outer skin layer (70).

5. Enriched air delivery device (1) according to claim 1, **characterized in that** the lower casing (20) is formed by two halves arranged relative to each other at a convergent angle (α), less than 180°.

6. Enriched air delivery device (1) according to claim 1, **characterized in that** the lower housing (20) has a U-shaped configuration, with two longitudinal side walls located in the vertical plane.

7. Enriched air delivery device (1) according to claim 6, **characterized in that** each of the two longitudinal side walls of the lower housing (20) has at least one ventilation grid (21).

8. Enriched air delivery device (1) according to claim 1, **characterized in that** it additionally comprises collagen lamps, located inside the device (1), between both housings (10, 20).

9. Enriched air delivery device (1) according to claim 1, **characterized in that** it additionally comprises heating means and/or vibration means, located inside the device (1), between both housings (10, 20), for stimulating the skin.

10. Enriched air delivery device (1) according to any one of the claims, **characterized in that** it has an ergonomic configuration, wherein both the upper casing (10) and the lower casing (20) have blunt and rounded edges and corners.

11. Enriched air delivery device (1) according to any one of the claims, **characterized in that** it can be attached to an extendable support (100), lamp type or the like.
